Europäisches Patentamt

European Patent Office

Office européen des brevets

(19)

(11) **EP 1 245 239 A1**

(12) **EUROPEAN PATENT APPLICATION**

(43) Date of publication:
02.10.2002 Bulletin 2002/40

(51) Int Cl.7: **A61L 15/60**

(21) Application number: **02252211.4**

(22) Date of filing: **27.03.2002**

(84) Designated Contracting States:
**AT BE CH CY DE DK ES FI FR GB GR IE IT LI LU
MC NL PT SE TR**
Designated Extension States:
**AL LT LV MK RO SI**

(30) Priority: **27.03.2001 GB 0107653**

(71) Applicant: **Bristol-Myers Squibb Company
New York, N.Y. 10154 (US)**

(72) Inventors:
• **Chen, Wai Yuen John
Wilmslow, Cheshire (GB)**

• **Moseley, Ryan
Maesteg, Mid-Glamorgan (GB)**
• **Waddington, Rachel Jane
Creigiau, Cardiff (GB)**
• **Walker, Michael
Brynsannan, Brynford, Flintshire (GB)**

(74) Representative: **Mays, Julie
Barker Brettell,
10-12 Priests Bridge
London SW15 5JE (GB)**

(54) **Wound dressing**

(57) Use of a polymer which is swellable in aqueous media for the manufacture of a wound dressing comprising the polymer to reduce the concentration of superoxide radical in a wound by application of the wound dressing externally thereto. This is believed to reduce the effect of at least certain types of ROS in frustrating the healing of chronic wounds.

EP 1 245 239 A1

## EP 1 245 239 A1

**Description**

This invention relates to the dressing of wounds; more particularly, the present invention relates to the dressing of chronic wounds using materials not customarily regarded as pharmaceutical agents.

It is known that, in chronic wounds, a large number of endogenously produced factors are released into the wound. It is believed by some workers in this field that the abnormalities of tissue repair demonstrated in chronic wounds may, at least in part, be due to the overactivity of tissue degradation mechanisms mediated by some of these factors. In particular, there is some evidence to suggest that reactive oxygen species (ROS), such as the superoxide radical ($O_2^{\cdot-}$) and the hydroxyl radical ($^\cdot OH$), derived from polymorphonuclear leukocytes may be implicated in the pathogenesis of chronic wounds. Thus, it is believed that the presence of ROS may result in depletion of cutaneous antioxidants in the wound and that excess ROS, produced in the wound, causes extensive extracellular matrix degradation to dermal components such as collagens, proteoglycans and hyaluronan. The reactive oxygen species may also affect the metabolism of cells responsible for the synthesis of such components.

This invention seeks to reduce the effect of at least certain types of ROS in frustrating the healing of chronic wounds.

According, therefore to one aspect of this invention, there is provided use of a polymer which is swellable in aqueous media for the manufacture of a wound dressing comprising the polymer to reduce the concentration of superoxide radical in a wound by application of the wound dressing externally thereto.

This invention also provides the use of a polymer as herein defined as a wound dressing agent.

This invention further provides a method of treatment of the human body, which method comprises applying externally to a wound on the body a wound dressing which comprises a polymer which is swellable in aqueous media, the application thereby reducing the concentration of superoxide radical in the wound.

In accordance with these aspects of the invention the polymer may be a substituted or unsubstituted, homo- or co-polysaccharide. Suitably, the polymer may comprise uronic acid groups. Desirably, the substituted polysaccharide may comprise etherified or acylated hydroxyl groups, and/or may comprise esterified uronic groups. In particular, the substituted polysaccharide may comprise at least some hydroxyl groups which have been replaced by amino or acylated amino groups.

The aforementioned substituent may comprise a saturated or unsaturated, carbocyclic or heterocyclic, mono or polycyclic group. The unsaturated group may include an aromatic group. Suitably, the polycyclic group may comprise a fused polycyclic structure.

It is preferred that the weight average molecular weight of the polymer may be from 200kDa to 2000kDa, preferably from 300kDa to 1000kDa. The polymer may comprise a substituted hyaluronan or a substituted cellulose. The polymer may be crosslinked. It may be formed as a film or as a fibre. Furthermore, a mixture of polymers as herein defined may be used in the manufacture of the wound dressing. When the polymer, or at least a component in a mixture of polymers, is formed as a fibre, the fibres may be disposed in the wound dressing manufactured therefrom as a non-woven mat or as a woven fibre. The materials in the wound dressing manufactured therefrom may be associated with one or more non-water swellable materials.

This invention also provides a method of treatment of the human body, which method comprises applying externally to a wound on the body a wound dressing which comprises a polymer selected from the group consisting of esterified hyaluronans, etherified celluloses and acylated celluloses, the polymer having a weight average molecular weight from about 200kDa to about 2000kDa, the application thereby reducing the concentration of superoxide radical in the wound.

While it is intended that the wound dressings in accordance with the present invention may be used without preservatives or pharmacologically active ingredients, it is possible to include these in minor amount. For example, an antibiotic or antimicrobial agent such as metronidazole, silver sulphadiazine, neomycin or penicillin; antiseptic agents such as povidone iodine; antiinflammatory agents such as hydrocortisone or triamcinolone acteonide; or skin protective agents such as zinc oxide may be included.

The following Figures illustrate the invention.

Figure 1 shows the inhibition of cytochrome C reduction by various materials in the presence of a high $O_2^{\cdot-}$ flux.

Figure 2 shows the inhibition of cytochrome C reduction by various materials in the presence of a medium $O_2^{\cdot-}$ flux.

Figure 3 shows the inhibition of cytochrome C reduction by various materials in the presence of a low $O_2^{\cdot-}$ flux.

Figure 4 shows the inhibition of cytcochrome C reduction by HYAFF-11™ and high molecular weight hyaluronan, by polymorphonuclear leukocyte-derived superoxide radicals

Figure 5 shows the degradation of 2-deoxy-D-ribose by a high $^\cdot OH$ flux in the presence of various materials.

Figure 6 shows the degradation of 2-deoxy-D-ribose by a medium $^\cdot OH$ flux in the presence of various materials.

Figure 7 shows the degradation of 2-deoxy-D-ribose by a low $^\cdot OH$ flux in the presence of various materials.

The following Examples illustrate the invention.

EXAMPLE 1

This Example compares the antioxidant abilities of different test materials in relation to superoxide radicals generated by cell-free systems. In the several runs of the Example, a flux of superoxide radicals was generated by oxidation of hypoxanthine by xanthine oxidase. The rate of production of the superoxide radical was measured by permitting it to reduce added cytochrome C. This reduction:

$$\text{Cytochrome C (Fe}^{+3}) + O_2^{\cdot-} \rightarrow \text{Cytochrome C (Fe}^{+2}) + O_2$$

produces a characteristic shift in the cytochrome C spectrum at 550 nm; and the rate of production of the superoxide radical can be determined spectrophotometrically, assuming the above stoichiometry, from the Beer-Lambert law and a molar extinction coefficient of 21,000 cm/moles/L. This is based on the assumption that one mole of radical reduces one mole of cytochrome C in accordance with the above equation. The abilities of the test materials to scavenge superoxide radicals can be determined from any decrease in the amount of reduced cytochrome C obtained in their presence.

In a total volume of 1ml of 50mM potassium phosphate buffer, pH 7.8, containing 10mM EDTA, three differing fluxes of superoxide radical were generated by the oxidation of hypoxanthine (ex Sigma) by xanthine oxidase (grade III from buttermilk, ex Sigma); these were:

| low | 1mM hypoxanthine and 1mU/ml xanthine oxidase |
| medium | 2mM hypoxanthine and 2mU/ml xanthine oxidase |
| high | 10mM hypoxanthine and 10mU/ml xanthine oxidas |

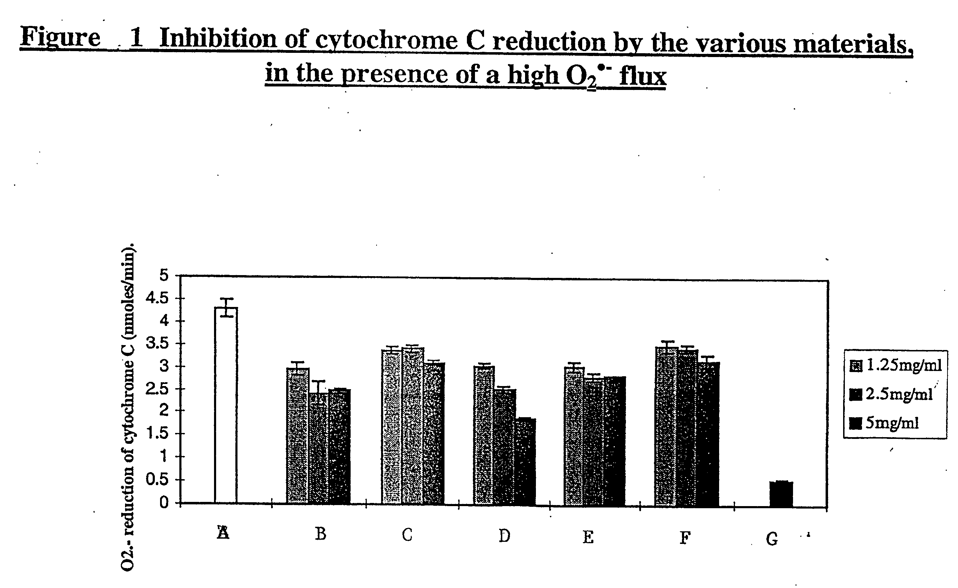

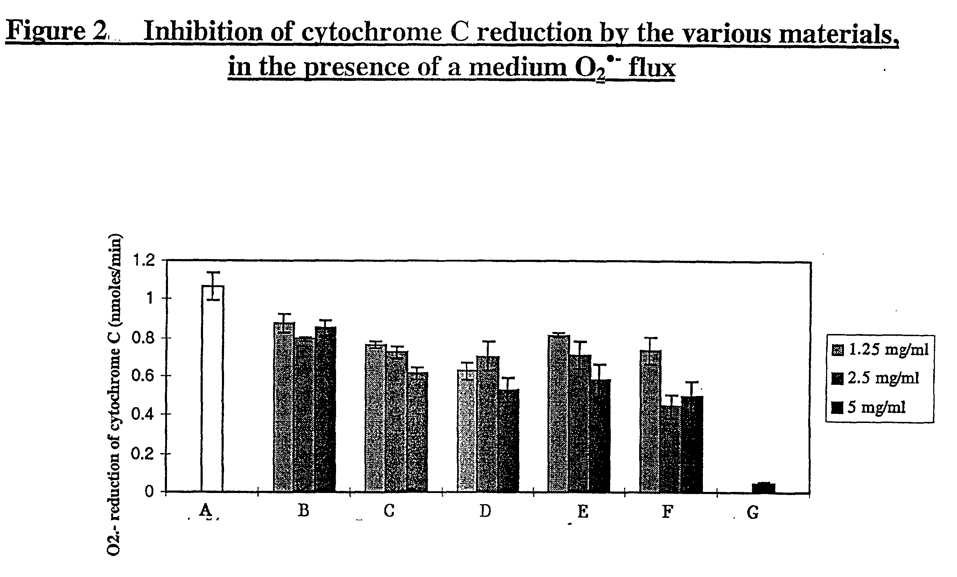

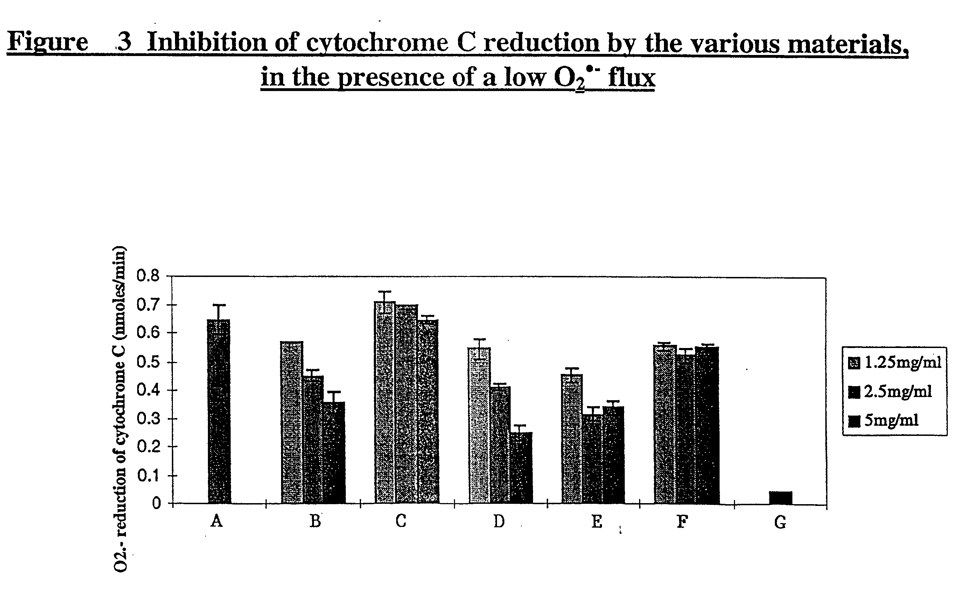

Each reaction mixture also contained 10μM cytochrome C (horse heart type III, ex Sigma).
To determine the antioxidant properties of each test material, at each of the abovementioned fluxes of superoxide radical, like reaction mixtures were next prepared but which also contained the test material, identified below, at concentrations of 1.25; 2.5 or 5mg/ml for each test material examined.
A control reaction mixture containing 40U/ml superoxide dismutase (SOD) (bovine erythrocytes, ex Sigma) was also established at each of the above-mentioned fluxes of superoxide radical.
Each experiment was performed in triplicate.
Upon initiation of the superoxide radical fluxes, the reduction of cytochrome C was monitored spectrophotometrically at room temperature, using a LKB Biochem Ultrospec plus 4054 UV/Visible spectrophotometer at 550nm (ex Amersham Pharmacia Biotech) against blank reaction mixtures containing all reagents except xanthine oxidase. The absorbance values were read at 20 sec intervals for 200 secs and the relative rates of production of the superoxide radical were calculated.
The results are shown in Figures 1; 2 and 3 in which the test materials are identified as follows:

A: control containing hypoxanthine and xanthine oxidase only;
B: high molecular weight hyaluronan (Mw∼3 to 6000kDa, human umbilical cord, ex Sigma);
C: low molecular weight hyaluronan (Mw∼300kDa, bovine vitreous humor, ex Sigma);
D: HYAFF-11 (65% by weight benzylated ester of hyaluronan, Mw∼300kDa);
E: carboxymethyl cellulose (Mw∼1000kDa, AQUACEL ex ConvaTec Ltd);
F: a 1% solution of high molecules weight hyaluronic acid (Mw∼IOOOkDa) placed onto E (Mw∼1000kDa, ex ConvaTec Ltd), thereby creating a thin film of approximately 500μm;
G: 40μl of SOD, a positive control.

HYAFF and AQUACEL are registered trade marks.
The result for each test material is in the form of bars showing the reduction in n moles/min at increasing concentration of test material. Thus for B in Figure 1 the left hand bar shows the reduction at 1.25mg/ml of test material, the central bar shows the reduction at 2.5mg/ml and the right hand bar shows the reduction at 5mg/ml.
It will be apparent that all of the test materials showed antioxidant behaviour toward the superoxide radical. This behaviour appears both molecular weight and dose dependent. Upon examination of the general trends of antioxidant activity of the test materials, D appeared to be the most effective towards the superoxide radical followed by E, B and F. All of these test materials exhibited greater antioxidant properties towards the superoxide radical than C.

The reduction of cytochrome C by the superoxide species was confirmed by the ability of SOD, at all of the fluxes of superoxide radical studied, extensively to reduce the rates of cytochrome C reduction b the superoxide radical; SOD is a known scavenger for the superoxide radical.

EXAMPLE 2

This Example compares the antioxidant abilities of certain of the test materials used in the last Example in relation to superoxide radicals generated by isolated polymorphonuclear leukocytes.

Whole blood (25ml) was removed from healthy human volunteers (age range 20 to 30 years) into vacutainers containing EDTA (ex Becton Dickinson, Meylan Cedex, France) as anticoagulant. Blood aliquots (2.5ml) were then layered onto a Ficoll-Hypaque density gradient which consisted of i) a dense Ficoll-Hypaque layer (2.5ml) comprising 9.5% by weight Ficoll 400 (ex Amersham Pharmacia Biotech) and 17% by weight Hypaque solution (sodium diatrizoate ex Sanofi Winthrop) and ii) a light Ficoll-Hypaque layer (2.5ml) comprising 8.17% by weight Ficoll 400 and 10% by weight Hypaque solution. Tubes were next centrifuged at 2500rpm for 45 minutes at room temperature and this treatment resulted in the formation of four layers. The two upper layers, consisting of plasma and lymphocytes/monocytes, respectively, were discarded. Each third layer (which contained the polymorphonuclear leukocytes) was removed and the several such third layers were pooled into a separate tube. The fourth layer (which comprised erythrocytes) was also discarded.

Equal volumes of phosphate buffered saline were added to the pooled polymorphonuclear leukocyte layers which were then centrifuged at 2000rpm for 5 minutes at room temperature. The centrifuging was repeated and the polymorphonuclear leukocyte pellet was resuspended in RPMI-1640 medium (phenol red free ex Gibco), supplemented with L-glutamine (2mM), prior to cell counting. After cell counting ($1 \times 10^6$ cells/ml), reaction mixtures were established by also including in a total volume of 1ml (1 mg/ml) cytochrome C (horse heart type 111, ex Sigma).

To determine the antioxidant properties of each test material like reaction mixtures (1 ml) were next prepared but which also included either material D or material B at concentrations of 0.5; 1.25; 2.5 or 5 mg/ml. A control reaction mixture containing 40U/ml SOD (bovine erythrocytes ex Sigma) was also established for each material.

1μg/ml N-formylmethionyteucyphenylatamine (FMLP) (ex Sigma) was then added to each reaction mixture to initiate the respirator burst. The reaction mixtures were next incubated at 37°C for 10 minutes and the reaction terminated by placing reaction mixtures on ice and centrifuging at 12500 rpm for 25 minutes at 4°C. The reduction of cytochrome C by polymorphonuclear leukocyte-derived superoxide radical was measured by analogy to the procedure in Example 1, blank reaction mixtures containing all reagents except FMLP.

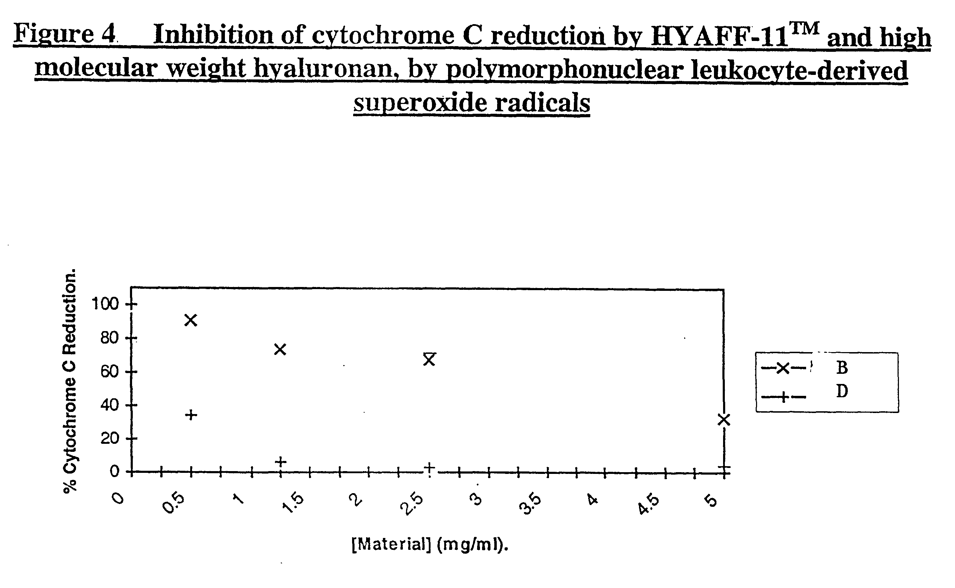

The results are shown in Figure 4. It will be apparent that both materials tested exhibited significant, dose dependent antioxidant activity towards the superoxide radical, with material D being the more effective.

COMPARATIVE EXAMPLE

This Example compares the antioxidant abilities of the same materials as were tested in the Examples of the invention in relation to hydroxyl radicals generated by cell-free systems. In the several runs of the comparative Example, a flux of hydroxyl radicals was generated by the Fenton reaction:

$Fe^{+2} + H_2O_2 \rightarrow {}^{\bullet}OH + OH^- + Fe^{+3}$

The rate of production of the hydroxyl radical was measured by permitting it to degrade 2-deoxy-D-ribose to malondialdehyde and then reacting the malondialdehyde with thio-barbituric acid, under acid conditions, to form a chromogen with an absorption maximum at 532 nm. This permits spectrophotometric determination essentially as before.

In a total volume of 1ml of 0. 1 M potassium phosphate buffer, pH 7.4, containing 0.15M sodium chloride, three differing fluxes of hydroxyl radical were generated by the reaction of $H_2O_2$, and $Fe^{+2}$; these were:

| low | 45μM $H_2O_2$ and 5μM $Fe_2SO_4$ |
| medium | 90μm $H_2O_2$ and 10μM $Fe_2SO_4$ |
| high | 180μM $H_2O_2$ and 20μM $Fe_2SO_4$ |

To determine the antioxidant properties of each test material, at each of the above-mentioned fluxes of hydroxyl radical, like reaction mixtures were next prepared but which also contained the test material, identified above, at concentrations of 0. 5; 1.25; 2.5 or 5mg/ml for each test material examined.

A control reaction mixture containing 10mM thiourea (ex Sigma was also established at each of the above-mentioned fluxes of hydroxyl radical: thiourea is a known scavenger of hydroxyl radical.

The reaction mixtures were then incubated at 37°C for 1 hour and centrifuged at 12500rpm for 10 minutes at

room temperature. 250μL alignots of each reaction mixture of each reaction mixture were next added to 2.8% trichloracetic acid (250μl) and 10% of thiobarbituric acid in 50mM sodium hydroxide (250μl). The assay mixtures were then heated at 100°C for 15 minutes and cooled.

Each experiment was performed in triplicate. The spectrophotometric determination was effect essentially as before against blank reaction mixtures containing all reagents thiobarbituric acid.

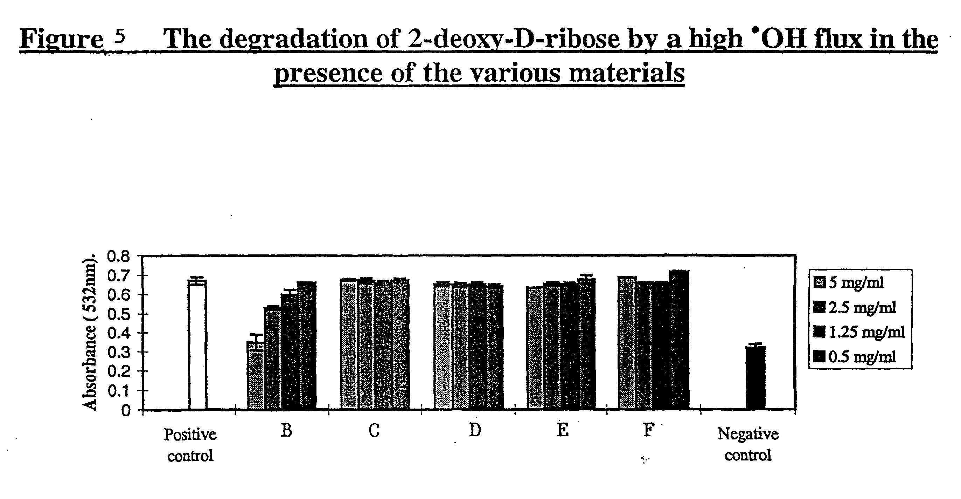

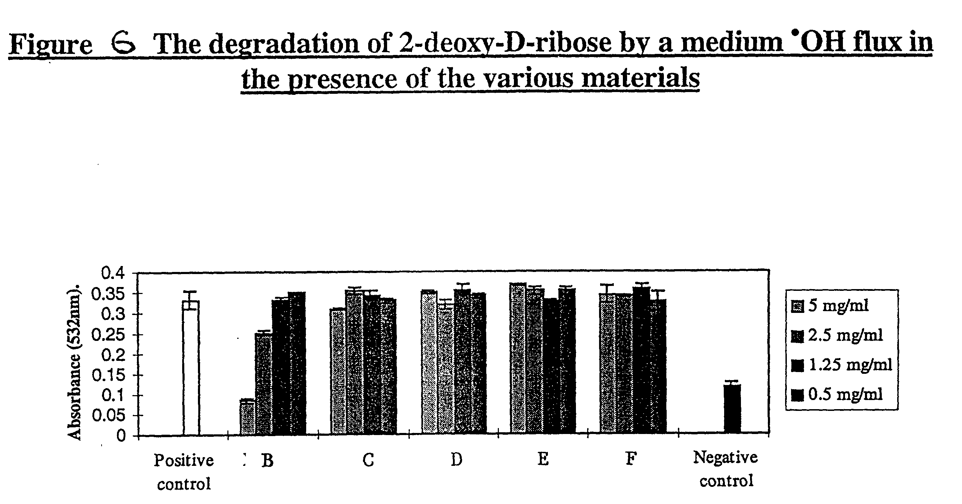

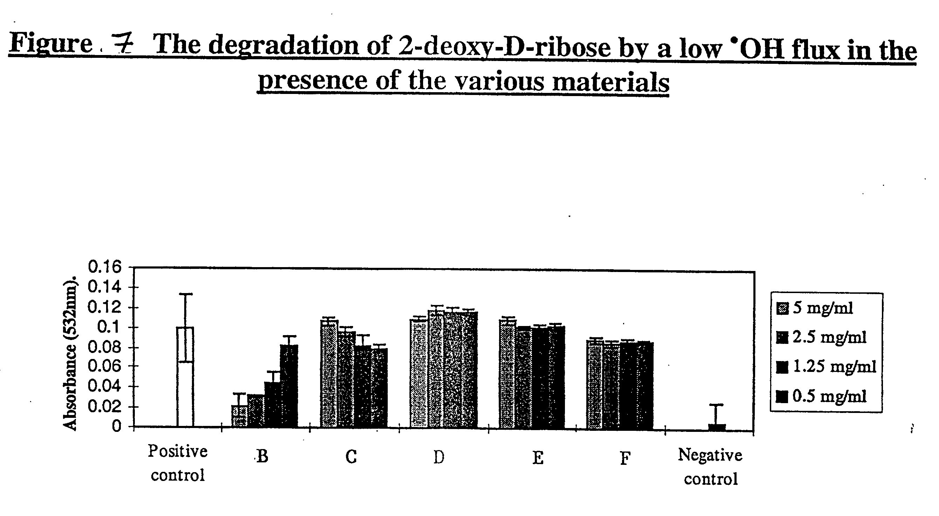

The results are shown in Figures 5; 6 and 7. The result for each test material is in the form of bars showing the degradation of 2-deoxy-D-ribose by hydroxyl radical at varying concentrations of the test material. Thus for B in Figure 5 the far left hand bar shows the degradation at 5mg/ml of test material, the concentration of test material decreasing so that the far right hand bar shows the degradation at 0.5mg/ml of test material.

It will be apparent from these results that the only material to possess significant, dose dependent antioxidant properties towards the hydroxyl radical is B.

It is clear from the results of these Examples that the effect shown for all materials (other than B) is highly specific to the superoxide radical.

## Claims

1.  Use of a polymer which is swellable in aqueous media for the manufacture of a wound dressing comprising the polymer to reduce the concentration of superoxide radical in a wound by application of the wound dressing externally thereto.

2.  Use according to claim 1 wherein the polymer is a substituted or unsubstituted, homo- or co-polysaccharide.

3.  Use according to claim 1 or 2 wherein the polymer comprises uronic acid groups.

4.  Use according to any preceding claim wherein the substituted polysaccharide comprises etherified or acylated hydroxyl groups.

5.  Use according to any preceding claim wherein the substituted polysaccharide comprises esterified uronic acid groups.

6.  Use according to any preceding claim wherein the substituted polysaccharide comprises at least some hydroxyl groups which have been replaced by amino or acylated amino groups.

7.  Use according to any preceding claim wherein the substituent comprises a saturated or unsaturated, carbocyclic or heterocyclic, mono or polycyclic group.

8.  Use according to claim 7 wherein the unsaturated group includes an aromatic group.

9.  Use according to claim 7 or 8 wherein the polycyclic group comprises a fused polycyclic structure.

10. Use according to any preceding claim wherein the weight average molecular weight of the polymer is from 200kDa to 2000kDa, preferably from 300kDa to 1000kDa

11. Use according to any preceding claim wherein the polymer comprises a substituted hyaluronan.

12. Use according to any preceding claim wherein the polymer comprises a substituted cellulose.

13. Use according to any preceding claim wherein the polymer is crosslinked.

14. Use according to any preceding claim wherein the polymer is formed as a film.

15. Use according to any preceding claim wherein the polymer is formed as a fibre.

16. Use according to any preceding claim wherein a mixture of polymers as defined in any of claims 1 to 13 is used in the manufacture of the wound dressing.

17. Use according to claim 15 wherein the fibres are disposed in the wound dressing manufactured therefrom as a non-woven mat or as a woven fibre.

18. Use according to any preceding claim wherein the materials in the wound dressing manufactured therefrom are associated with one or more non-water swellable materials.

19. Use of a polymer according to any of claims 1 to 16 as a wound dressing agent.

20. A method of treatment of the human body, which method comprises applying externally to a wound on the body a wound dressing which comprises a polymer which is swellable in aqueous media, the application thereby reducing the concentration of superoxide radical in the wound.

21. A method according to claim 20 wherein in polymer is defined in any one of claims 1 to 16.

22. A method of treatment of the human body, which method comprises applying externally to a wound on the body a wound dressing which comprises a polymer selected from the group consisting of esterified hyaluronans, etherified celluloses and acylated celluloses, the polymer having a weight average molecular weight from about 200kDa to about 2000kDa, the application thereby reducing the concentration of superoxide radical in the wound.

Figure 1 Inhibition of cytochrome C reduction by the various materials, in the presence of a high $O_2^{\bullet-}$ flux

O2.- reduction of cytochrome C (nmoles/min).

Legend: 1.25mg/ml, 2.5mg/ml, 5mg/ml

EP 1 245 239 A1

## Figure 2. Inhibition of cytochrome C reduction by the various materials, in the presence of a medium $O_2^{\bullet-}$ flux

8

## Figure 3 Inhibition of cytochrome C reduction by the various materials, in the presence of a low $O_2^{\bullet-}$ flux

Legend:
- 1.25mg/ml
- 2.5mg/ml
- 5mg/ml

Y-axis: $O_2^{\bullet-}$ reduction of cytochrome C (nmoles/min)

X-axis: A, B, C, D, E, F, G

# Figure 4 Inhibition of cytochrome C reduction by HYAFF-11™ and high molecular weight hyaluronan, by polymorphonuclear leukocyte-derived superoxide radicals

EP 1 245 239 A1

**Figure 5    The degradation of 2-deoxy-D-ribose by a high •OH flux in the presence of the various materials**

EP 1 245 239 A1

Figure 6  The degradation of 2-deoxy-D-ribose by a medium ·OH flux in the presence of the various materials

Legend:
- 5 mg/ml
- 2.5 mg/ml
- 1.25 mg/ml
- 0.5 mg/ml

Y-axis: Absorbance (532nm).

X-axis categories: Positive control, B, C, D, E, F, Negative control

Figure 7 The degradation of 2-deoxy-D-ribose by a low •OH flux in the presence of the various materials

## PARTIAL EUROPEAN SEARCH REPORT

Application Number

which under Rule 45 of the European Patent Convention shall be considered, for the purposes of subsequent proceedings, as the European search report

EP 02 25 2211

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | FOSCHI D ET AL: "HYALURONIC ACID PREVENTS OXYGEN FREE-RADICAL DAMAGE TO GRANULATION TISSUE: A STUDY IN RATS" INTERNATIONAL JOURNAL OF TISSUE REACTIONS, BIOSCIENCE EDIPRINT, GENEVA, CH, vol. 12, no. 6, 1990, pages 333-339, XP000953074 ISSN: 0250-0868 * abstract * * page 334, left-hand column * * page 338, right-hand column, last paragraph * | 1-3,5-7, 10,11, 14,19 | A61L15/60 |
| X | US 5 667 501 A (BURROW THOMAS RICHARD ET AL) 16 September 1997 (1997-09-16) * column 1, line 20 - line 38 * * column 2, line 22 - line 56 * * figures 1,4-6 * * claims * | 1,2,4,6, 7,13,17, 19 | |

-/--

TECHNICAL FIELDS SEARCHED (Int.Cl.7)

A61L

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

Although claims 20-22 are directed to a method of treatment of the human/animal body (Article 52(4) EPC), the search has been carried out and based on the alleged effects of the compound/composition.

| Place of search | Date of completion of the search | Examiner |
|---|---|---|
| THE HAGUE | 5 July 2002 | Muñoz, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

EP 1 245 239 A1

| European Patent Office | PARTIAL EUROPEAN SEARCH REPORT | Application Number |
|---|---|---|
| | | EP 02 25 2211 |

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| X | WO 01 00190 A (CALLEGARO LANFRANCO ;DALLE CARBONARE MAURIZIO (IT); FIDIA ADVANCED) 4 January 2001 (2001-01-04) * column 1, line 20 - line 38 * * column 2, line 22 - line 56 * * examples 1,4-6 * | 1-11,13, 14,16, 17,19 | |
| X | WO 94 16746 A (BURROW THOMAS RICHARD ;COURTAULDS PLC (GB); BAHIA HARDEV SINGH (GB) 4 August 1994 (1994-08-04) * page 3, line 22 - line 27 * * page 11, line 8 - line 32 * * claims 1,12,13 * | 1,2,4, 12,13, 15-19 | |
| A | PRESTI D ET AL: "Hyaluronan-mediated protective effect against cell damage caused by enzymatically produced hydroxyl (OH.) radicals is dependent on hyaluronan molecular mass." CELL BIOCHEMISTRY AND FUNCTION. ENGLAND DEC 1994, vol. 12, no. 4, December 1994 (1994-12), pages 281-288, XP008005344 ISSN: 0263-6484 * abstract * | | TECHNICAL FIELDS SEARCHED (Int.Cl.7) |
| T | MOSELEY R ET AL: "Comparison of the antioxidant properties of HYAFFR)-11p75, AQUACEL) and hyaluronan towards reactive oxygen species in vitro" BIOMATERIALS, ELSEVIER SCIENCE PUBLISHERS BV., BARKING, GB, vol. 23, no. 10, May 2002 (2002-05), pages 2255-2264, XP004348222 ISSN: 0142-9612 | 1-22 | |

EPO FORM 1503 03.82 (P04C10)

15

## ANNEX TO THE EUROPEAN SEARCH REPORT
## ON EUROPEAN PATENT APPLICATION NO.

EP 02 25 2211

This annex lists the patent family members relating to the patent documents cited in the above-mentioned European search report.
The members are as contained in the European Patent Office EDP file on
The European Patent Office is in no way liable for these particulars which are merely given for the purpose of information.

05-07-2002

| Patent document cited in search report | | Publication date | Patent family member(s) | | Publication date |
|---|---|---|---|---|---|
| US 5667501 | A | 16-09-1997 | AT | 191348 T | 15-04-2000 |
| | | | AU | 679954 B2 | 17-07-1997 |
| | | | AU | 5654694 A | 04-07-1994 |
| | | | DE | 69328308 D1 | 11-05-2000 |
| | | | EP | 0673262 A1 | 27-09-1995 |
| | | | GB | 2288736 A ,B | 01-11-1995 |
| | | | JP | 8503869 T | 30-04-1996 |
| WO 0100190 | A | 04-01-2001 | IT | PD990144 A1 | 29-12-2000 |
| | | | AU | 6559800 A | 31-01-2001 |
| | | | WO | 0100190 A2 | 04-01-2001 |
| | | | EP | 1196179 A2 | 17-04-2002 |
| WO 9416746 | A | 04-08-1994 | WO | 9416746 A1 | 04-08-1994 |
| | | | AU | 680863 B2 | 14-08-1997 |
| | | | AU | 5863394 A | 15-08-1994 |
| | | | BR | 9406261 A | 30-01-1996 |
| | | | DE | 69409363 D1 | 07-05-1998 |
| | | | DE | 69409363 T2 | 08-10-1998 |
| | | | DK | 680344 T3 | 11-01-1999 |
| | | | EP | 0680344 A1 | 08-11-1995 |
| | | | JP | 8505790 T | 25-06-1996 |
| | | | RU | 2135212 C1 | 27-08-1999 |
| | | | SK | 92695 A3 | 07-02-1996 |
| | | | US | 6075177 A | 13-06-2000 |
| | | | AT | 164523 T | 15-04-1998 |
| | | | CA | 2154473 A1 | 04-08-1994 |
| | | | CZ | 9501827 A3 | 17-01-1996 |
| | | | ES | 2115929 T3 | 01-07-1998 |
| | | | NZ | 259734 A | 27-07-1997 |

EPO FORM P0459

For more details about this annex : see Official Journal of the European Patent Office, No. 12/82